Europäisches Patentamt

European Patent Office

Office européen des brevets

(11) Publication number: **0 299 532 B1**

# EUROPEAN PATENT SPECIFICATION

(45) Date of publication of patent specification: **23.09.92**

(51) Int. Cl.5: **A61F 13/15**

(21) Application number: **88111495.3**

(22) Date of filing: **15.07.88**

(54) **Sanitary napkin with disposal means.**

(30) Priority: **16.07.87 US 75020**

(43) Date of publication of application:
**18.01.89 Bulletin 89/03**

(45) Publication of the grant of the patent:
**23.09.92 Bulletin 92/39**

(84) Designated Contracting States:
**AT BE CH DE ES FR GB IT LI LU NL**

(56) References cited:
**EP-A- 0 121 966**
**DE-A- 3 121 390**
**US-A- 3 973 567**
**US-A- 4 608 047**

(73) Proprietor: **McNEIL-PPC, INC.**
**Van Liew Avenue**
**Milltown New Jersey 08850(US)**

(72) Inventor: **Seidy, Wassim**
**211 Haworth Place**
**Somerset New Jersey 08873(US)**

(74) Representative: **Strehl, Schübel-Hopf, Groening**
**Maximilianstrasse 54 Postfach 22 14 55**
**W-8000 München 22(DE)**

Rank Xerox (UK) Business Services

## Description

### Field of the Invention

This invention relates to protective, absorbent liners for undergarments, especially those that provide their own means for disposal.

### Background of the Invention

Conventional sanitary napkins generally comprise an absorbent core having a liquid pervious layer on the body facing side and a liquid impervious layer on the undergarment facing side. Typically, these napkins comprise non-woven fabrics and/or plastic film components which are not readily dispersed in water so that the soiled napkin cannot be disposed of merely by flushing away in a water closet. Accordingly, napkin users are faced with the task of disposing of used napkins in open waste paper baskets, and often resort to wrapping the soiled napkin in tissue before discarding.

Prior art attempts to provide for the disposal of soiled napkins have not, in the main, proved to be entirely successful. Self-contained bags affixed to the garment facing side of the napkin have been employed by manufacturers, but these can interfere with the adhesive attachment sites and are associated with increased material and manufacturing costs. See U.S. Pat. No. 4,182,336. Other approaches have included the use of adhesive means disposed on the sealed ends of the napkin, whereby when the napkin is rolled onto its body-facing side to form a cylinder, the adhesive means are employed to seal the napkin. See U.S. Pat. No. 3,626,945. This later approach has met with little success since the user usually must touch the soiled body-facing side of the napkin when it is being rolled. Moreover, the rolling action can compress the absorbent material and express the absorbed fluids from the exposed sides of the napkin. Another solution to the disposal problem is addressed in Baum, U.S. Pat. No. 4,402,689, which provides a baffle or fluid impermeable surface that may be folded over the body-facing side of the napkin where it is adhesively attached. Although this product provides for disposing the napkin without the user having to touch the soiled napkin surface, the napkin remains in a fully extended position and may not be discreetly deposited into a waste basket.

In a more recent patent by Mattingly, U.S. Patent No. 4.608,047, a napkin is disclosed which may be folded about two transverse fold lines for disposing. Mattingly describes a newer generation napkin which provides side flaps for overlying the outer crotch portion of an undergarment.

US-A- 4 608 047 discloses a sanitary napkin for placement in the inner crotch of an undergarment comprising:

    (a) an elongated central absorbent element having two ends, two longitudinal edges, an undergarment facing side and a body facing side;

    (b) a backing layer substantially covering the undergarment-facing side of the absorbent element;

    (c) at least one flap extending transversely beyond the longitudinal edge of the backing layer; and

    (d) adhesive means disposed on the backing layer;

    (e) further adhesive means on the flaps.

The flaps of Mattingly are not designed to be merely an impervious backing material, but are preferably body fluid absorbing. Because of the absorbing nature of these flaps, they may contain body fluid and, accordingly, can complicate the handling and disposal of the napkin. Moreover, because Mattingly is limited to only one securing means and requires that the napkin be folded into three segments prior to being wrapped within the flap portions, napkin users may find this disposal solution to be inconvenient.

Accordingly, there is a need for a sanitary napkin comprising its own convenient disposal means which can be manufactured without significant additional cost. There is also a need for a napkin that can be sealed for disposal and folded into a compact size without leaking.

It is, therefore, an object of this invention to provide a sanitary napkin having its own disposal means which can be manufactured without significant additional cost.

It is another object of this invention to provide adhesive means on a sanitary napkin which serve the dual purpose of providing a means for attachment of the napkin to an undergarment and a means for securing the napkin in a folded configuration for disposal.

With these and other object in view, which will become apparent to one skilled in the art as the description proceeds, this invention resides in the novel construction, combination, arrangement of parts and methods substantially as hereinafter described and more particularly defined by the attached claims.

These objects are achieved according to claims 1 and 6. Preferred embodiments are subject-matter of the dependant claims. These objects are achieved according to claims 1 to 6. Preferred embodiments are subject-matter of the dependant claims.

### Summary of the Invention

A novel sanitary napkin is provided which is capable of being folded and self-sealed for disposal. This napkin includes a backing layer having at least one flap which extends transversely from the longitudinal edge at one end of the napkin. Upon folding the napkin for disposal, the flap of the backing layer is wrapped around the folded portion of the napkin to engage appropriately positioned adhesive means on the other end of the backing layer to effectively enclose the soiled portions of the napkin and keep the absorbed body fluid from leaking. The napkin is designed so that it can be folded onto its body-facing surface about a transverse axis to provide a compact size prior to disposal. The cost of this additional feature is minimal since only a small piece of additional impervious backing layer material is required. Accordingly, a novel napkin is provided which comprises its own convenient, compact disposal means.

In the preferred embodiment of this invention, two flaps are provided extending from opposing longitudinal edges at one end of the napkin. The flaps are conveniently folded back over and detachably secured to the undergarment facing side of the backing layer during use. Each of these folded over flaps has pressure sensitive adhesive means on its exposed surface which contact the undergarment during use. The adhesive means also secure the flaps to the undergarment facing portion of the backing layer once the napkin is folded upon its body-facing side and the flaps are wrapped around the folded portion of the napkin prior to disposal. Pressure sensitive adhesive means are also provided on the other end of the backing layer away from said flaps for adhering the backing layer to the undergarment during use, and for adhering to the flaps when the napkin is folded and the flaps are wrapped around for disposal. Accordingly, in this embodiment each adhesive means serves a dual purpose of adhering a portion of the backing layer to the undergarment during use, and adhering appropriate portions of the backing layer to each other during disposal. In a preferred embodiment, the adhesive means on the flaps and the adhesive means on the other end of the backing layer are positioned so that they contact one another for maximum security of attachment when the flaps are wrapped around the folded napkin.

The present invention also provides a novel method for disposing of a sanitary napkin comprising the steps of rolling or folding and sealing the napkin using one or more self-contained transverse flaps extending from one end of the backing layer. In accordance with this method, the body facing side of the napkin is folded upon itself along a transverse axis and the transverse flap or flaps are unfolded from their position of use against the backing layer and wrapped around the folded portion of the napkin and adhered to undergarment facing portions of the backing layer on the other end of the napkin. In accordance with this method, adhesive means provided on each flap and on the other end of the backing layer are used to adhere the flaps and the backing layer to the undergarment during use, and to each other when the napkin is folded for disposal.

Brief Description of the Drawings

The accompanying drawings illustrate a complete embodiment of the invention according to the best mode so far devised for the practical application of the principles thereof, and in which:
FIG. 1: is a perspective view from the garment-facing side of the napkin illustrating a preferred embodiment with two flaps and four adhesive means with their protective release papers being partially removed;
FIG. 2: is a perspective view of the napkin in FIG. 1 illustrating how the flaps may be extended transversely;
FIG. 3: is a perspective view of the napkin after it has been folded onto its body-facing side illustrating how the flaps may be folded around the napkin and how the adhesive means may be adhered to seal the napkin;
FIG. 4: is a top planar view of a fully sealed napkin which is shown partially cut away to illustrate how the adhesive means have been adhered to seal the flaps;
FIG. 5: is a cross-sectional view of the napkin of FIG. 4, taken on the line 5-5 thereof.

Detailed Description of the Invention

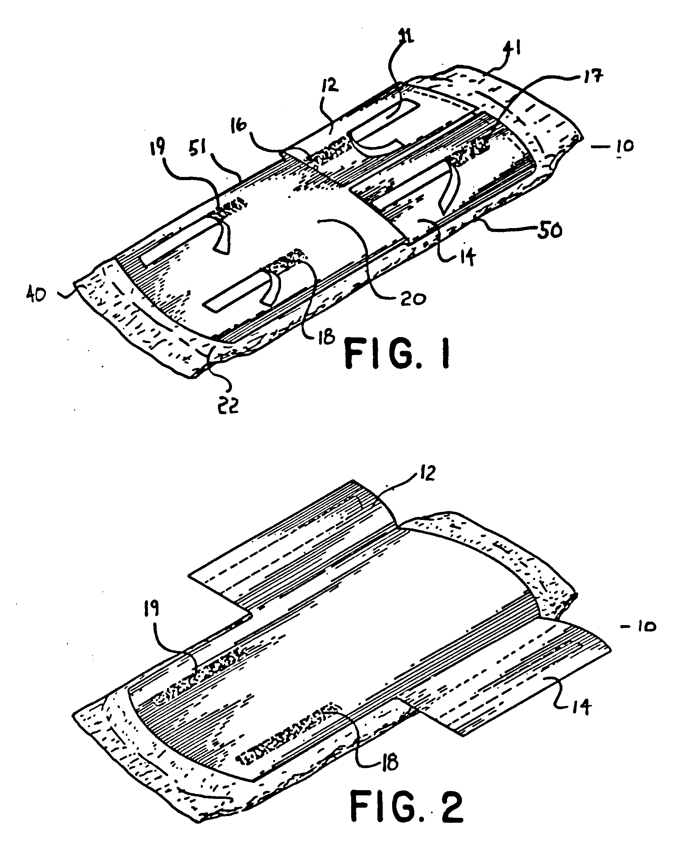

With reference to the drawings, and particularly FIGS. 1-3 thereof, there is shown a preferred sanitary napkin 10 having a body-facing side and an undergarment-facing side. The napkin 10 comprises an elongated central absorbent 22 having two ends 40 and 41, two longitudinal edges 50 and 51, an undergarment-facing side and a body-facing side. Provided on the undergarment-facing side of the absorbent 22 is a backing layer 20, preferably a body-fluid impervious layer. The backing layer 20 is provided with a first adhesive means, depicted as a pair of adhesive strips 18 and 19, which ideally are disposed on the undergarment facing side of the backing layer 20. Extending transversely from one "end" of the backing layer 20 is at least one flap which is adapted to be folded over backing layer 20 and which preferably has its own pressure-sensitive adhesive means on the exposed surface when folded. For the purpose of describing the backing layer 20, the term "end" refers to that portion of said layer defined by a transverse section line taken at about the midpoint of the backing layer's longitudinal side. In a more preferred embodiment of this invention, a sanitary napkin 10 comprises at least two flaps, shown in FIG. 1 as flaps 12 and 14. These flaps extend transversely from the opposing longitudinal edge over the full length of one end of the backing layer 20, and each flap has its own pressure-sensitive adhesive means 16 and 17 on the exposed surface when said flaps are folded over backing layer 20.

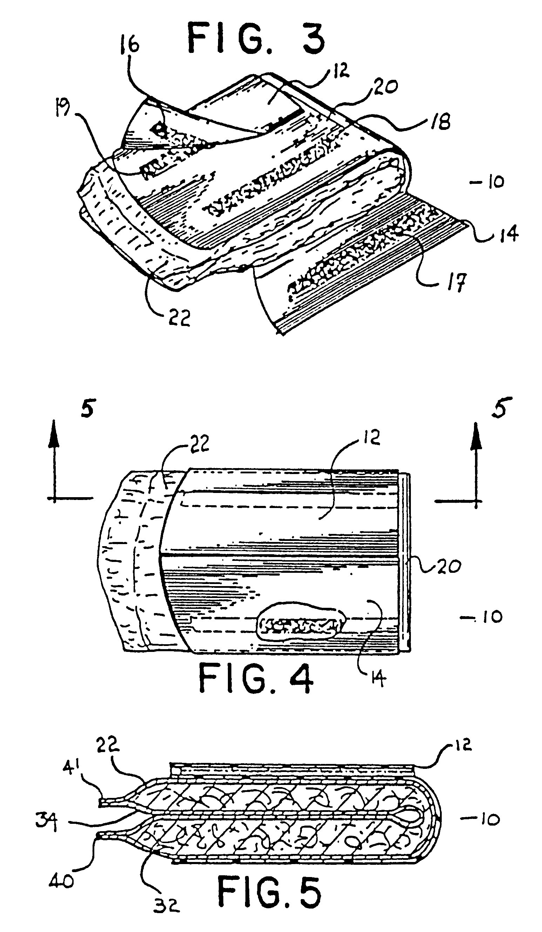

In both the above embodiments, the flap or flaps are preferably of sufficient transverse length to overlap a folded portion of said napkin 10 after the napkin has been folded about a transverse axis for disposal. In the one-flap embodiment, the transverse length should be greater than in the two-flap embodiment to provide enough material to overlap the folded portion of said napkin 10, and is preferably substantially equal to the width of the napkin. In the two-flap embodiment of FIG. 3, it is preferred that the flaps have a transverse length of about one half the width of the napkin, although this is not a requirement, and this dimension can vary depending on the location of the adhesive means and the degree of overlap required.

In the preferred embodiment of the present invention, the flap or flaps have a longitudinal length approximately equal to one half the length of the napkins, and the napkin is folded in half about a transverse axis at the midpoint of the napkin for sealing and disposal.

The adhesive means on the flaps and on backing layer 20 is preferably arranged so that when the napkin 10 is folded about a transverse axis as illustrated in FIG. 3, the first adhesive means on the backing layer, shown as adhesive strips 18 and 19, contacts the adhesive means on the flaps, shown in FIG. 3 as adhesive strip 16 and 17. It is also anticipated that, in either the one-flap or two-flap embodiment of this invention, when the flap or flaps are in the folded position on the garment-facing side of the napkin, they are removably secured to the body-fluid impervious backing layer prior to use. Preferably, the flap or flaps are ultrasonically or adhesively secured to the backing layer so that they remain in a folded position during attachment but can easily be separated from the backing layer when the napkin is folded for disposal.

The preferred method of this invention initially provides an elongated central absorbent 22 having two transverse ends 40 and 41, two longitudinal edges 50 and 51, an undergarment-facing side and a body-facing side. The central absorbent 22 is covered by a backing layer or body-fluid impervious layer 20 on the undergarment-facing side of the napkin. Backing layer 20 preferably is selected to include at least one flap extending transversely from an end of said layer with means disposed thereon to adhere at least said flap to said backing layer 20. First pressure-sensitive adhesive means are disposed on an undergarment facing side of the backing layer 20 and on the exposed surface of said flap when said flap is adhered to said backing layer. The method proceeds to fold a portion of napkin 10 about a transverse axis onto its body-facing side. The flap or flaps are then released from the backing layer, wrapped around the folded portion of the napkin, and secured to the other side of said backing layer 20 using said first pressure-sensitive adhesive means, thereby sealing the napkin 10 for disposal.

In a most preferred method of this invention, the backing or body-fluid impervious layer 20 is selected to comprise at least two flaps 12 and 14 extending transversely from opposing longitudinal edge of the barrier layer at one end of the napkin. Each of these flaps is selected to have pressure-sensitive adhesive means 16 and 17 on the exposed surface when said flaps are folded over backing layer 20.

The choice of materials for use in the napkin 10 of this invention may be any of the well-known absorbent, super-absorbent, woven and non-woven materials and adhesives utilized in the art of manufacturing these products. The absorbent element 22 of this invention should be made of soft, comfortable material. Adequate absorbency may be built into the core 32 of the absorbent without adding bulk by adding super-absorbent materials, now known, which have the properties of high-liquid retention, e.g. cross-linked acrylate polymers. In a preferred embodiment of this invention, the absorbent element 22 contains conventional resilient materials, e.g., staple-length synthetic fibers, for maintaining the bulk and absorbent capacity of the napkin. Generally, the absorbent element 22 should be about 10,16 to 25,4 cm (4-10 inches) in length, preferably about 15,24 to 22,86 cm (6-9 inches) and is folded approximately in half at the time of disposal.

As described in FIG. 5, the absorbent element 22 comprises a core 32 which preferably is made of loosely associated absorbent hydrophilic material such as cellulose fibers, wood pulp, regenerated cellulose or cotton fibers, and/or other materials generally known in the art. Such fibers may be chemically or physically modified and the core may include such fibers in combination with other materials, both natural and synthetic such as hydrophilic foams, hydrophilic polymers or the like. For the preferred embodiment of this invention, wood pulp is the material of the choice because of its availability and inexpensive cost.

As is customary in the art, the side of the napkin to be worn against the body of the user is covered with a body fluid pervious layer 34. This surface may be any liquid pervious woven or non-woven material or perforated plastic film. Preferably, body facing material should be a material which readily allows the passage of fluid while retaining little or no fluid in its structure to provide a relatively dry surface next to the skin. Generally, the fluid permeable surface 34 is a single, rectangular sheet of material having a width sufficient to cover the body-facing side of the absorbing element 22.

The body-fluid impervious backing layer 20 is preferably made from fluid impermeable material such as polyethylene or a non-woven material coated with an impermeable film. The impervious layer should preferably allow the passage of air and moisture vapor while substantially blocking the passage of liquids. The impervious layer 20 in the preferred embodiment is sealed together with the body facing layer 34 around the perimeter of the absorbent element 22 to prevent leakage of fluid from the sides of the absorbent element 22. The impervious layer 20 may be heated or fastened by way of adhesives to the core 32 or to the core 32 wrapped in a pervious layer cover 34. The fluid impervious layer 20 is generally fastened to the core 32 by means of a plurality of longitudinally extending lines of adhesive. Preferably, however, the impervious layer 20 is heat bondable material such as polyethylene, which may be bonded to the pervious layer 34 to completely enclose the core 32.

In the preferred embodiment of this invention, the impervious layer 20 is provided with at least one integral flap extending transversely from a longitudinal edge at one end thereof which may be overlapped around the napkin after it has been folded onto its body-facing surface to seal the napkin for disposal. In a particularly preferred embodiment of this invention, the impervious layer 20 comprises at least two flaps and which extend transversely from opposing edges at one longitudinal end of the napkin 10. Ideally each of these flaps has its own adhesive means which serve as a means for attaching the napkin to an undergarment and also as a means for adhering the flaps to the impervious layer after the napkin is folded for disposal. As shown in FIG. 4, the adhesive means on the flaps and backing layer of the more preferred embodiment are arranged so that they overlap one another when the flaps are folded around the napkin. In this embodiment, adhesive means 16 on flap 12 is aligned with adhesive means 19 on backing layer 20 and adhesive means 17 on flap 14 is aligned with adhesive means 18 on backing layer 20 to secure the flaps 12 and 14 in the position for napkin disposal as depicted in FIGS. 3 and 4. In an alternate embodiment, the flap or flaps are not integral with the backing layer but are obtained by securing a separate piece of material, preferably a liquid impervious material, to the backing layer so that the ends of the material extend transversely beyond the longitudinal edges of the backing material.

The adhesive materials used for the sanitary napkin of this invention should be made of any known pressure-sensitive adhesive materials suited for the purposes of this invention. Compositions suitable for sanitary napkins include, for example, the water-based, pressure-sensitive adhesives such as the acrylate adhesives, e.g., vinyl acetate-2 ethyl hexyl acetate copolymer which is generally combined with tackifiers such as, for example, ethylene amine. Alternatively, the adhesive may comprise the rapid setting thermoplastic hot-melt adhesives. The adhesive elements may also comprise a two-sided adhesive tape or materials based on an elastomer selected from natural or synthetic rubbers. In the illustrated preferred embodiment of this invention, four adhesive means 16, 17, 18, and 19 are utilized for the dual purpose of securing the napkin to the undergarment during use and for adhering the flaps 12 and 14 to the impervious layer 20 after the napkin 10 has been folded for disposal. As is customary in the art of sanitary napkins, the adhesive means are covered by a release paper material, i.e., strip 11 in FIG. 1, which protects the adhesive surface until the napkin is ready for attachment to the undergarment.

The release paper means of this invention may be made of any sheet-like material which adheres with sufficient tenacity to the adhesive means 16, 17, 18 and 19 to remain in place, but which can be readily removed when the napkin 10 is to be used. Conventional materials used for this purpose include woven webs, non-woven bonded fiber webs, non-woven threaded webs, threaded reinforced non-woven webs, plastic films, and/or laminates of the above. A particularly useful material for release paper is a semi-bleached kraft paper, the adhesive contacting side of which has been silicone-coated to provide easier removal from the adhesive means.

From the foregoing, it can be realized that this invention provides an improved sanitary napkin with its own convenient disposal means. The napkin can be manufactured inexpensively and only requires a small piece of additional impervious material to form the flap or flaps on its garment facing side. Accordingly, this invention provides a cleaner and more discreet way of sealing the napkin for disposal and satisfies a need which has, heretofore not been addressed by prior art products. Although various embodiments have been illustrated, this was for the purpose of describing, but not limiting the invention. Various modifications, which will become apparent to one skilled in the art, are within the scope of this invention described in the attached claims.

## Claims

1. A sanitary napkin capable of being folded or rolled and self-sealed for disposal comprising:

   (a) an elongated central absorbent element (22) having two ends (40, 41), two longitudinal edges (50, 51), an undergarment facing side and a body facing side;

   (b) a backing layer (20) substantially covering the undergarment-facing side of the absorbent element (22);

   (c) at least one flap (12, 14) extending transversely beyond the longitudinal edge of the backing layer (20);

   (d) first adhesive means (18, 19) provided on the backing layer (20); and

   (e) second adhesive means (16, 17) provided on the flap(s) (12,14);

   **characterized in that** that said at least one flap (12, 14), being adapted to be folded over the backing layer (20), is disposed in one end portion of the backing layer (20); said end portion is defined by a longitudinal section between a transverse section line taken at about the midpoint of the backing layer (20) and one (41) of the ends of said elongated central absorbent element (22); the second adhesive means (16, 17) are disposed on the exposed surface of said folded over flap (12, 14) and contact the first adhesive means (18, 19) of said backing layer (20) when the napkin is rolled or folded about a transverse axis for disposal and said flap (12, 14) is wrapped around the rolled or folded napkin.

2. The sanitary napkin according to claim 1, characterized in that the backing layer (20) comprises a liquid impervious layer.

3. The sanitary napkin according to claim 1 or 2, characterized in that said flap(s) (12, 14) is an integral part of said backing layer (20).

4. The sanitary napkin according to one of claims 1 to 3, characterized in that the first adhesive means (18,19) is disposed on the undergarment facing side of the backing layer (20) at the half portion thereof not occupied by the flap(s) (12, 14).

5. The sanitary napkin according to one of claims 1 to 4, characterized in that the flap(s) (12, 14) is folded over the half portion of the backing layer (20) and detachably secured thereto.

6. A method for self-sealing a sanitary napkin for disposal by folding a napkin (10) comprising an elongated central absorbent (22) having two ends (40, 41), two longitudinal edges (50, 51), an undergarment facing side and a body facing side, and a backing layer (20) on said undergarment facing side with at least one flap (12, 14) extending transversely beyond a longitudinal edge of said backing layer (20), said backing layer (20) having pressure-sensitive first adhesive means (18, 19) disposed thereon and the flap(s) having second adhesive means (16, 17) provided thereon,

   **characterized in that**

   a) at least one flap (12, 14), which is adapted to be folded over the backing layer (20), is provided in one end portion of the backing layer (20), said end portion being defined by a longitudinal section between a transverse section line taken at about the midpoint of the backing layer (20) and one (41) of the ends of said elongated central absorbent element (22), and the second adhesive means (16, 17) are disposed on the exposed surface of the folded over flaps (12, 14);

   b) folding the napkin (10) about a transverse axis;

   c) wrapping the flap(s) (12, 14) around the folded portion of the napkin (10);

   d) adhering the flap(s) (12, 14) to said backing layer (20) using the pressure-sensitive first adhesive means (18, 19) of the backing layer contacting the second adhesive means (16, 17) on the exposed surface of the flap(s), thereby sealing the sanitary napkin (10) for disposal.

7. The method according to claim 6, characterized in that the first adhesive means (18, 19) is disposed on the garment facing side of the backing layer (20) at the half portion thereof not occupied by the flap(s).

8. The method according to claim 6 or 7, characterized in that the flap(s) (12, 14) is folded over the half portion of the backing layer (20) and detachably secured thereto prior to use.

## Patentansprüche

1. Damenbinde, die zum Wegwerfen gefaltet oder gerollt und selbstklebend sein kann, mit

   (a) einem länglichen, mittleren, absorbierenden Element (22) mit zwei Enden (40, 41), zwei Längsseiten (50, 51), einer der Unterwäsche zugewandten Seite und einer dem Körper zugewandten Seite;

(b) einer Rückschicht (20), die im wesentlichen die der Unterwäsche zugewandte Seite des absorbierenden Elements (22) bedeckt;

(c) mindestens einem Lappen (12, 14), der sich quer über die Längsseite der Rückschicht (20) erstreckt;

(d) ersten Klebmitteln (18, 19), die auf der Rückschicht (20) vorgesehen sind; und

(e) zweiten Klebmitteln (16, 17), die auf dem (den) Lappen (12, 14) vorgesehen sind;

**dadurch gekennzeichnet, daß**

der genannte mindestens eine Lappen (12, 14), der über die Rückschicht (20) faltebar ist, in einem Endteil der Rückschicht (20) angebracht ist; der genannte Endteil durch einen sich längs erstreckenden Abschnitt zwischen einer Querschnittslinie definiert ist, die etwa beim Mittelpunkt der Rückschicht (20), und einem (41) der Enden des genannten länglichen mittleren absorbierenden Elements (22) verläuft; die zweiten Klebmittel (16, 17) auf der frei liegenden Oberfläche des genannten gefalteten Lappens (12, 14) angebracht sind und mit den ersten Klebmitteln (18, 19) der genannten Rückschicht (20) in Berührung sind, wenn die Binde zum Wegwerfen entlang einer Querachse gerollt oder gefaltet ist und der genannte Lappen (12, 14) die gerollte oder gefaltete Binde umhüllt.

2. Damenbinde nach Anspruch 1, dadurch gekennzeichnet, daß die Rückschicht (20) eine für Flüssigkeiten undurchlässige Schicht ist.

3. Damenbinde nach Anspruch 1 oder 2, dadurch gekennzeichnet, daß der (die) genannte(n) Lappen (12, 14) ein wesentlicher Bestandteil der genannten Rückschicht (20) ist (sind).

4. Damenbinde nach einem der Ansprüche 1 bis 3, dadurch gekennzeichnet, daß die ersten Klebmittel (18, 19) auf der der Unterwäsche zugewandten Seite der Rückschicht (20) in der Hälfte angebracht sind, die nicht von dem (den) Lappen (12, 14) besetzt ist.

5. Damenbinde nach einem der Ansprüche 1 bis 4, dadurch gekennzeichnet, daß der (die) Lappen (12, 14) über die Hälfte der Rückschicht (20) gefaltet und daran abtrennbar befestigt ist.

6. Verfahren zum Selbstverkleben einer Damenbinde zum Wegwerfen durch Falten einer Binde (10), mit einem länglichen, mittleren, absorbierenden Element (22) mit zwei Enden (40, 41), zwei Längsseiten (50, 51), einer der Unterwäsche zugewandten Seite und einer dem Körper zugewandten Seite, einer Rückschicht (20) auf der der Unterwäsche zugewandten Seite mit mindestens einem Lappen (12, 14), der sich quer über eine Längsseite der genannten Rückschicht (20) erstreckt, wobei die genannte Rückschicht (20) darauf angebrachte druckempfindliche erste Klebmittel (18, 19) aufweist, und der (die) Lappen darauf angebrachte zweite Klebmittel (16, 17) hat (haben),

**dadurch gekennzeichnet, daß**

a) mindestens ein Lappen (12, 14), der über die Rückschicht (20) faltbar ist, in einem Endteil der Rückschicht (20) angebracht wird, wobei der genannte Endteil durch einen Längsabschnitt zwischen einer Querschnittslinie definiert ist, die etwa beim Mittelpunkt der Rückschicht (20), und einem (41) der Enden des genannten länglichen mittleren absorbierenden Elements (22) verläuft, und die zweiten Klebmitteln (16, 17) auf der frei liegenden Oberfläche des genannten gefalteten Lappens (12, 14) angebracht sind;

b) die Binde (10) um eine Querachse gefaltet wird;

c) der (die) Lappen (12, 14) um den gefalteten Teil der Binde (10) gelegt wird (werden);

d) der (die) Lappen (12, 14) an die genannte Rückschicht (20) geklebt werden unter Verwendung der druckempfindlichen ersten Klebmittel (18, 19) der Rückschicht, die mit den zweiten Klebmitteln (16, 17) auf der frei liegenden Oberfläche des (der) Lappen(s) in Berührung sind, wodurch die Binde (10) zum Wegwerfen verschlossen ist.

7. Verfahren nach Anspruch 6, dadurch gekennzeichnet, daß die ersten Klebmittel (18, 19) auf der der Unterwäsche zugewandten Seite der Rückschicht (20) in der Hälfte angebracht werden, die nicht von dem (den) Lappen besetzt ist (sind).

8. Verfahren nach Anspruch 6 oder 7, dadurch gekennzeichnet, daß der (die) Lappen (12, 14) über die Hälfte der Rückschicht (20) gefaltet und daran vor der Verwendung abtrennbar befestigt wird (werden).

**Revendications**

1. Une serviette hygiénique pouvant être pliée ou roulée et auto-collée afin d'être jetée comprenant :

(a) un élément absorbant central allongé (22) ayant deux extrémités (40, 41), deux bords longitudinaux (50, 51) un côté faisant face au sousvêtement et un côté faisant face au corps ;

(b) une couche de recouvrement (20) recouvrant substantiellement le côté faisant face au sous-vêtement de l'élément absorbant (22) ;

(c) au moins un rabat (12, 14) s'étendant transversalement au delà du bord longitudinal de la couche de recouvrement (20) ;

(d) des premiers moyens adhésifs (18, 19) fournis sur la couche de recouvrement (20) ; et

(e) des seconds moyens adhésifs (16, 17) fournis sur le (les) rabat(s) (12, 14) ;

caractérisée en ce que ledit au moins un rabat (12, 14), qui est adapté pour être plié sur la couche de recouvrement (20), est disposé à une partie d'extrémité de la couche de recouvrement (20) ; ladite partie d'extrémité est définie par une coupe longitudinale entre une ligne de coupe transversale prise à peu près au point central de la couche de recouvrement (20) et une extrémité (41) dudit élément central absorbant allongé (22) ; les seconds moyens adhésifs (16, 17) sont disposés sur la surface exposée dudit rabat replié (12, 14) et sont en contact avec les premiers moyens adhésifs (18, 19) de ladite couche de recouvrement (20) lorsque la serviette est roulée ou pliée le long de l'axe transversal afin d'être jetée, et ledit rabat (12, 14) est enveloppé autour de la serviette roulée ou pliée.

2. La serviette hygiénique selon la revendication 1, caractérisé en ce que la couche de recouvrement (20) comprend une couche imperméable aux liquides.

3. La serviette hygiénique selon la revendication 1 ou 2, caractérisée en ce que ledit (lesdits) rabat(s) (12, 14) fait (font) partie intégrale de ladite couche de recouvrement (20).

4. La serviette hygiénique selon l'une des revendications 1 à 3, caractérisée en ce que les premiers moyens adhésifs (18, 19) sont disposés sur le côté faisant face au sous-vêtement de la couche de recouvrement (20) au niveau de la moitié de celle-ci qui n'est pas occupée par le (les) rabat(s) (12, 14).

5. La serviette hygiénique selon l'une des revendications 1 à 4, caractérisée en ce que le (les) rabat(s) (12, 14) est (sont) pliés sur la moitié de la couche de recouvrement (20) et y est (sont) fixé(s) de telle sorte qu'on puisse l'en (les en) détacher.

6. Un procédé pour auto-coller une serviette hygiénique afin de la jeter en pliant une serviette (10) comprenant un élément absorbant central allongé (22) ayant deux extrémités (40, 41), deux bords longitudinaux (50, 51), un côté faisant face au sous-vêtement et un côté faisant face au corps, et une couche de recouvrement (20) sur ledit côté faisant face au sous-vêtement avec au moins un rabat (12, 14) s'étendant transversalement au delà d'un bord longitudinal de ladite couche de recouvrement (20), ladite couche de recouvrement (20) ayant des premiers moyens adhésifs sensibles à la pression (18, 19) qui y sont disposés, et le (les) rabat(s) étant muni(s) de seconds moyens adhésifs (16, 17), caractérisé en ce que :

(a) au moins un rabat (12, 14), qui est adapté pour être plié sur la couche de recouvrement (20), est fourni au niveau d'au moins une extrémité de la couche de recouvrement (20), ladite partie d'extrémité étant définie par une coupe longitudinale entre une ligne de coupe transversale prise à peu près au point central de la couche de recouvrement (20) et une extrémité (41) dudit élément central absorbant allongé (22) et les seconds moyens adhésifs (16, 17) étant disposés sur la surface exposée du rabat replié (12, 14) ;

(b) la serviette (10) est pliée le long d'un axe transversal ;

(c) le (les) rabat(s) (12, 14) est (sont) plié(s) le long de la partie pliée de la serviette (10) ;

(d) le (les) rabat(s) (12, 14)est (sont) collé(s) à ladite couche de recouvrement (20) en utilisant les premiers moyens adhésifs sensibles à la pression (18, 19) de la couche de recouvrement qui est en contact avec les seconds moyens adhésifs (16, 17) situés sur la surface exposée du (des) rabat(s), ce qui permet de coller la serviette hygiénique (10) afin de la jeter.

7. Le procédé selon la revendication 6, caractérisé en ce que les premiers moyens adhésifs (18, 19) sont disposés sur le côté faisant face au vêtement de la couche de recouvrement (20) au niveau de la moitié de celle-ci qui n'est pas occupé par le (les) rabat(s).

8. Le procédé selon la revendication 6 ou 7, caractérisé en ce que le (les) rabat(s) (12, 14) est (sont) plié(s) sur la moitié de la couche de recouvrement (20) et y est (sont) fixé(s) de telle sorte qu'on puisse l'en (les en) détacher avant l'utilisation.

FIG. 1

FIG. 2

# FIG. 3

# FIG. 4

# FIG. 5